# EUROPEAN PATENT APPLICATION

(11) **EP 2 684 581 A1**
(43) Date of publication of application: **15.01.2014**
(21) Application number: 13175403.8
(22) Date of filing: 05.07.2013
(51) Int. Cl.: A61N 7/02

(54) **Method and apparatus for generating irradiation plan to be provided to focused ultrasound irradiation apparatus**

(30) Priority: 10.07.2012 KR 20120075178
(71) Applicant: Samsung Electronics Co., Ltd, Gyeonggi-do 443-742 (KR)
(72) Inventor: Ahn, Min-su, Gyeonggi-do (KR); Bang, Won-chul, Gyeonggi-do (KR)
(74) Representative: Hylarides, Paul Jacques

(57) **Abstract**

Methods and apparatuses for generating irradiation plans are provided. The method includes determining a size of a focal spot in an area, determining locations of focal spots in the area to which ultrasounds are to be sequentially irradiated based on the size of the focal spot, such that each of the locations of the focal spots is farthest from locations of focal spots in the area to which ultrasounds have been previously-irradiated, determining durations and intensities of ultrasound irradiations based on the size of the focal spot and the locations of the focal spots, and generating the irradiation plan for irradiating ultrasound based on the size of the focal spot, the locations of the focal spots, and the durations and the intensities of the ultrasound irradiations.

## Description

### BACKGROUND

### 1. Field

The following description relates to methods and apparatuses for generating irradiation plans to be provided to focused ultrasound irradiation apparatuses.

### 2. Description of the Related Art

As a non-invasive tumor removal technique, high intensity focused ultrasound (HIFU) irradiation is widely-used due to harmlessness to the human body. The HIFU is an irradiation method of inducing necrosis of lesional tissues by focusing and irradiating high intensity ultrasound onto lesions in a human body. The ultrasound focused and irradiated onto the lesions is transformed to thermal energy, to thereby raise a temperature at an irradiated portion to which the ultrasound is irradiated, and to thus induce coagulative necrosis of tissues and blood vessels at the irradiated portion. The temperature rises momentarily, and thus only the irradiated portion may be removed while preventing heat from spreading out of the irradiated portion.

### SUMMARY

In one general aspect, there is provided a method of generating an irradiation plan for irradiating ultrasound, the method including determining a size of a focal spot in an area to which ultrasound is to be irradiated. The method further includes determining locations of focal spots in the area to which ultrasounds are to be sequentially irradiated based on the size of the focal spot, such that each of the locations of the focal spots is farthest from locations of focal spots in the area to which ultrasounds have been previously-irradiated. The method further includes determining durations and intensities of ultrasound irradiations based on the size of the focal spot and the locations of the focal spots, and generating the irradiation plan for irradiating ultrasound based on the size of the focal spot, the locations of the focal spots, and the durations and the intensities of the ultrasound irradiations. Accordingly, an influence of a thermal dose due to a previous irradiation of ultrasound may be reduced or minimized.

In another general aspect, there is provided an irradiation plan generating apparatus configured to generate a irradiation plan for irradiating ultrasound, the irradiation plan generating apparatus including a first determining unit configured to determine a size of a focal spot in an area to which ultrasound is to be irradiated. The apparatus further includes a second determining unit configured to determine locations of focal spots in the area to which ultrasounds are to be sequentially irradiated based on the size of the focal spot, such that each of the locations of the focal spots is farthest from locations of focal spots in the area to which ultrasounds have been previously-irradiated. The apparatus further includes a third determining unit configured to determine durations and intensities of ultrasound irradiations based on the size of the focal spot and the locations of the focal spots, and a plan generating unit configured to generate an irradiation plan for irradiating ultrasound based on the size of the focal spot, the locations of the focal spots, and the durations and the intensities of the ultrasound irradiations. Accordingly, cooling time intervals between sequential irradiations of the ultrasounds may be reduced or minimized, and an overall operation time of the focused ultrasound irradiation process may be reduced.

In still another general aspect, there is provided a focused ultrasound irradiation apparatus including an image generating unit configured to generate an image of an area to which ultrasound is to be irradiated. The apparatus further includes a processor configured to determine a size of a focal spot in the area to which ultrasound is to be irradiated, determine locations of focal spots in the area to which ultrasounds are to be sequentially irradiated based on the size of the focal spot, such that each of the locations of the focal spots is farthest from locations of focal spots in the area to which ultrasounds have been previously-irradiated, and determine durations and intensities of ultrasound irradiations based on the size of the focal spot and the locations of the focal spots. The apparatus further includes a transducer configured to irradiate ultrasound based on the size of the focal spot, the locations of the focal spots, the durations and the intensities of the ultrasound irradiations included in an irradiation plan, and the image of the area.

In yet another general aspect, there is provided a focused ultrasound irradiation method including generating an image of an area to which ultrasound is to be irradiated. The method further includes determining a size of a focal spot in the area to which ultrasound is to be irradiated, determining locations of focal spots in the area to which ultrasounds are to be sequentially irradiated based on the size of the focal spot, such that each of the locations of the focal spots is farthest from locations of focal spots in the area to which ultrasounds have been previously-irradiated, and determining durations and intensities of ultrasound irradiations based on the size of the focal spot and the locations of the focal spots. The method further includes irradiating ultrasound based on the size of the focal spot, the locations of the focal spots, the durations and the intensities of the ultrasound irradiations included in a irradiation plan, and the image of the area.

Other features and aspects may be apparent from the following detailed description, the drawings, and the claims.

### BRIEF DESCRIPTION OF THE DRAWINGS

FIG. 1 is a block diagram illustrating an example of an irradiation plan generating apparatus configured to generate irradiation plans to be provided to a focused ultrasound irradiation apparatus.
FIG. 2 is a block diagram illustrating an example of a focused ultrasound irradiation apparatus.
FIG. 3 is a flowchart view illustrating an example of a method of generating irradiation plans to be provided to a focused ultrasound irradiation apparatus.
FIG. 4 is a flowchart view illustrating an example of a focused ultrasound irradiation method.

Throughout the drawings and the detailed description, unless otherwise described, the same drawing reference numerals will be understood to refer to the same elements, features, and structures. The relative size and depiction of these elements may be exaggerated for clarity, illustration, and convenience.

### DETAILED DESCRIPTION

The following detailed description is provided to assist the reader in gaining a comprehensive understanding of the methods, apparatuses, and/or systems described herein. Accordingly, various changes, modifications, and equivalents of the systems, apparatuses and/or methods described herein will be suggested to those of ordinary skill in the art. Also, descriptions of well-known functions and constructions may be omitted for increased clarity and conciseness.

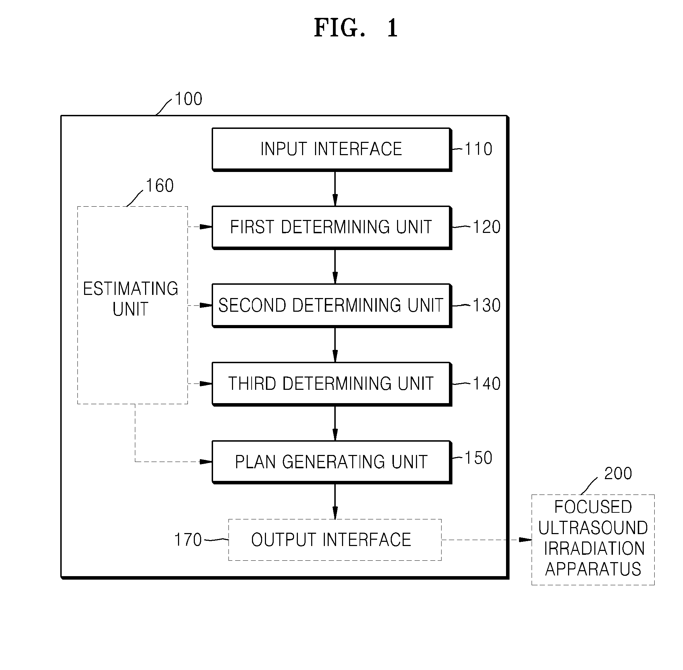

FIG. 1 is a block diagram illustrating an example of an irradiation plan generating apparatus 100 configured to generate irradiation. Referring to FIG. 1, the irradiation plan generating apparatus 100 includes an input interface 110, a first determining unit 120, a second determining unit 130, a third determining unit 140, and a plan generating unit 150. The irradiation plan generating apparatus 100 may further include an estimating unit 160 and an output interface 170.

The components of the irradiation plan generating apparatus 100 are only components related to the example of FIG. 1. Therefore, one of ordinary skill in the art will understand that components other than the components shown in FIG. 1 may further be included.

The irradiation plan generating apparatus 100 may correspond to or include at least one processor. Therefore, the irradiation plan generating apparatus 100 may be included in a computer system and operated by the computer system.

The irradiation plan generating apparatus 100 generates an irradiation plan to treat a lesion of a patient. The irradiation plan may be provided to the focused ultrasound irradiation apparatus 200. The irradiation plan indicates electronic data regarding a process of irradiating ultrasound to the lesion of the patient, in the focused ultrasound irradiation apparatus 200. Thus, the irradiation plan generating apparatus 100 generates electronic data representative the irradiation plan. The electronic data may be in various formats. Before the focused ultrasound irradiation apparatus 200 performs this process, the irradiation plan generating apparatus 100 determines whether or not a focused ultrasound irradiation is applicable to the lesion of the patient, and determines the method of irradiating the ultrasound to the lesion of the patient, to thereby generate the irradiation plan.

The focused ultrasound irradiation apparatus 200 treats a lesion of a patient via focused irradiation of ultrasound to the lesion of the patient. For example, the focused ultrasound irradiation apparatus 200 may use high intensity focused ultrasound (HIFU). For convenience of explanation, it will be assumed below that the focused ultrasound irradiation apparatus 200 uses HIFU. However, ultrasound that may be used by the focused ultrasound irradiation apparatus 200 is not limited thereto, and one of ordinary skill in the art will understand that any other types of focused ultrasound similar to HIFU may also be used by the focused ultrasound irradiation apparatus 200.

The input interface 110 receives, as an input, a target area to which ultrasound is to be irradiated. The target area corresponds to a lesion of a patient to be treated, and includes an area treatable by sequentially irradiating a single ultrasound during a single operation of the focused ultrasound irradiation apparatus 200. A size of the target area is limited. Thus, if a size of the lesion of the patient is larger than that of the target area, the focused ultrasound irradiation apparatus 200 may divide the lesion into a plurality of target areas to treat the lesion.

The irradiation plan generating apparatus 100 may receive a predetermined target area from an external device via the input interface 110, or may receive a lesion of a patient to be treated via the input interface 110 and determine a target area based on the lesion by itself. For convenience of explanation, it is assumed below that the irradiation plan generating apparatus 100 receives the predetermined target area from the external device via the input interface 110.

The target area may be displayed based on an image of the target area that is obtained via a computer tomography (CT) apparatus, a magnetic resonance imaging (MRI) apparatus, and/or a diagnostic ultrasound system. The irradiation plan generating apparatus 100 may compare images obtained via the CT apparatus, MRI apparatus, and/or diagnostic ultrasound system to each other, and analyze these images to obtain a more precise image of the target area.

The first determining unit 120 determines a size of a focal spot in a target area to which a single ultrasound is to be irradiated by the focused ultrasound irradiation apparatus 200. In more detail, the focused ultrasound irradiation apparatus 200 focuses on a topical portion of the target area, and intensively irradiates ultrasound thereto. In other words, ultrasound is irradiated to an area including the same size as the focal spot that is determined by the first determining unit 120. Tissues at a position of the focal spot to which the ultrasound is irradiated are destroyed or necrosed, and thus, a lesion is cured.

The irradiation plan generating apparatus 100 determines locations of focal spots in a target area, and durations and intensities of ultrasound to be irradiated by the focused ultrasound irradiation apparatus 200, based on the determined size of the focal spot. In this regard, the first determining unit 120 outputs the determined size of the focal spot to the second determining unit 130.

Based on the size of the focal spot determined by the first determining unit 120, the second determining unit 130 determines locations of focal spots in a target area to which ultrasounds are to be sequentially irradiated, such that each of the locations of the focal spots is the farthest from locations of focal spots in the target area to which ultrasounds have been previously-irradiated. For example, the second determining unit 130 determines a location of a first focal spot in a target area to which ultrasound is to be initially-irradiated, and a location of a second focal spot in the target area to which ultrasound is to be irradiated after the first focal spot, such that the location of the second focal spot is located the farthest from the location of the first focal spot. The second determining unit 130 may determine a location of a third focal spot in the target area to which ultrasound is to be irradiated after the second focal spot, such that the location of the third focal spot is located the farthest from the locations of the first and second focal spots.

When the second determining unit 130 determines locations of focal spots in a target area to which ultrasounds are to be irradiated, a location that is the farthest from locations of focal spots in the target area to which ultrasounds have been previously-irradiated, may be an area in which tissues are necrosed or do not correspond to a lesion. In this example, the second determining unit 130 determines another location that is the farthest from the locations of the focal spots to which ultrasounds have been previously-irradiated. In other words, when it is difficult to secure a sufficient distance for cooling from the locations of the focal spots to which the ultrasounds have been previously-irradiated, ultrasound is irradiated to the other farthest location after a sufficient cooling time interval.

Since the location of the second focal spot is the farthest from the location of the first focal spot, an influence of a thermal dose of the ultrasound irradiated to the first focal spot upon the second focal spot may be reduced or minimized. Furthermore, since the third focal spot is located the farthest from the locations of the first and second focal spots, an influence of a thermal dose of the ultrasound irradiated to the first and second focal spots upon the third focal spot may be reduced or minimized. Therefore, a cooling time interval between the irradiation of the ultrasound to the first focal spot and the irradiation of the ultrasound to the second focal spot, and a cooling time interval between the irradiation of the ultrasound to the second focal spot and the irradiation of the ultrasound to the third focal spot, may be reduced or minimized.

he cooling time intervals between the irradiations of the ultrasounds prevent normal tissues from being destroyed by a thermal dose accumulated in the target area due to the sequential irradiations of the ultrasounds to the target area. The cooling time intervals also cool heat accumulated in the target area due to a previous irradiation of ultrasound between irradiations of ultrasounds. Therefore, a cooling time interval is inversely proportional to a distance between two focal spots to which ultrasounds are sequentially irradiated.

According to a focused ultrasound irradiation process in the related art, locations of focal spots to which ultrasounds are sequentially irradiated are made to be as close to each other as possible. Therefore, after ultrasound is irradiated to each focal spot, temperatures of tissues may rise quickly at each focal spot, and thus, a HIFU operation time is reduced. In the related art, since temperatures of tissues rise quickly at a next focal spot to which ultrasound is irradiated, time intervals between sequential irradiations of ultrasounds may be reduced. However, as temperatures rise quickly, normal tissues to be protected may be necrosed.

Since the irradiation plan generating apparatus 100 plans to sequentially irradiate ultrasounds to focal spots that are arranged to be away from each other as far as possible, an influence of a thermal dose due to a previous irradiation of ultrasound may be reduced or minimized. Thus, cooling time intervals between sequential irradiations of the ultrasounds may be reduced or minimized during an overall focused ultrasound irradiation process. Furthermore, normal tissues may be prevented from being necrosed by the thermal dose due to the previous irradiation of the ultrasound. As a result, an overall operation time of the focused ultrasound irradiation process may be reduced, and the focused ultrasound irradiation operation may be performed in consideration of safety and convenience of a patient.

The irradiation plan generating apparatus 100 determines durations and intensities of ultrasound irradiations based on the determined size of the focal spot and the determined locations of the focal spots. Therefore, the second determining unit 130 outputs the size of the focal spot that is determined by the first determining unit 120, and the determined locations of the focal spots, to the third determining unit 140.

The third determining unit 140 determines durations and intensities of ultrasound irradiations based on the size of the focal spot and the locations of the focal spots. In other words, the third determining unit 140 determines a duration and an intensity of an ultrasound irradiation to each of the locations of the focal spots that are determined by the second determining unit 130 with the size of the focal spot that is determined by the first determining unit 120. For example, the third determining unit 140 may determine a duration and an intensity of an ultrasound irradiation to a location of a focal spot based on whether tissues outside a target area will be protected, and whether tissues at the focal spot to which ultrasound is to be irradiated may be necrosed.

The plan generating unit 150 generates an irradiation plan to irradiate ultrasound based on the size of the focal spot that is determined by the first determining unit 120, the locations of the focal spots that are determined by the second determining unit 130, and the durations and the intensities of the ultrasound irradiations that are determined by the third determining unit 140. The irradiation plan indicates a method of irradiating the ultrasound to a lesion of a patient, in the focused ultrasound irradiation apparatus 200. The irradiation plan may be generated as a computer executable program.

For example, based on the size of the focal spot, the locations of the focal spots, and the durations and the intensities of the ultrasound irradiations, the plan generating unit 150 may generate the irradiation plan to include information regarding a path in which a transducer of the focused ultrasound irradiation apparatus 200 is to move via, e.g., simulation. Furthermore, the plan generating unit 150 may generate the irradiation plan to include information regarding ultrasound to be irradiated by the transducer, e.g., a duration and an intensity of the ultrasound.

The estimating unit 160 estimates thermal doses and temperatures of a target area and focal spots in the target area to which ultrasounds are to be sequentially irradiated. The second determining unit 130 may determine the locations of the focal spots based on the size of the focal spot that is determined by the first determining unit 120, and the temperatures and thermal doses that are estimated by the estimating unit 160, such that each of the locations of the focal spots is the farthest from locations of focal spots in the target area to which ultrasounds have been previously-irradiated. For example, the second determining unit 130 may refer to the thermal doses and the temperatures to determine the locations of the focal spots based on whether tissues outside the target area will be protected and whether tissues at a focal spot in the target area to which ultrasound is to be irradiated may be necrosed.

The output interface 170 outputs an irradiation plan generated by the plan generating unit 150 to the focused ultrasound irradiation apparatus 200.

Although FIG. 1 shows that the irradiation plan generating apparatus 100 includes the estimating unit 160 and the output interface 170, the irradiation plan generating apparatus 100 is not limited thereto, and the irradiation plan generating apparatus 100 may operate without including the estimating unit 160 and the output interface 170. Therefore, the estimating unit 160 and the output interface 170 may not be components of the irradiation plan generating apparatus 100.

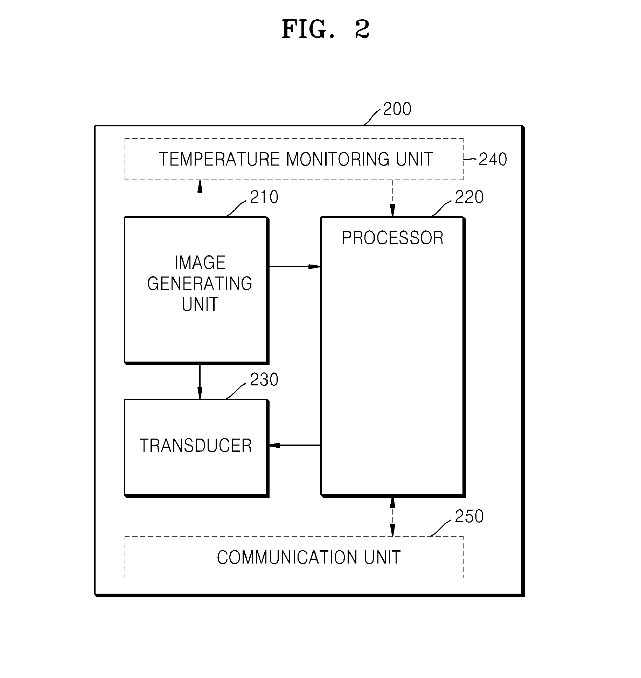

FIG. 2 is a block diagram illustrating an example of the focused ultrasound irradiation apparatus 200. Referring to FIG. 2, the focused ultrasound irradiation apparatus 200 includes an image generating unit 210, a processor 220, and a transducer 230. The focused ultrasound irradiation apparatus 200 may further include a temperature monitoring unit 240 and a communication unit 250.

The components of the focused ultrasound irradiation apparatus 200 are only components related to the example of FIG. 2. Therefore, one of ordinary skill in the art will understand that components other than the components shown in FIG. 2 may further be arranged.

The image generating unit 210 generates an image of a target area to which ultrasound is to be irradiated. The target area corresponds to a lesion of a patient to be treated, and includes an area treatable by sequentially irradiating a single ultrasound during a single operation of the focused ultrasound irradiation apparatus 200. The image generating unit 210 may generate the image of the target area by using a CT apparatus, an MRI apparatus, and/or a diagnostic ultrasound system. However, the example of FIG. 2 is not limited thereto.

Furthermore, the image generating unit 210 may monitor an irradiation process of a target area in real time. For example, the image generating unit 210 may monitor whether normal tissues are protected, and whether tissues at a focal spot in the target area to which ultrasound is irradiated are necrosed. Furthermore, the image generating unit 210 may obtain images of the target area, and determine whether the irradiation process is completed.

The processor 220 determines a size of a focal spot in a target area to which a single ultrasound is to be irradiated. The processor 220 also determines locations of focal spots in the target area to which ultrasounds are to be sequentially irradiated, based on the determined size of the focal spot, such that each of the locations of the focal spots is the farthest from locations of focal spots to which ultrasounds have been previously-irradiated. The processor 220 further determines durations and intensities of ultrasound irradiations based on the determined size of the focal spot and the determined locations of the focal spots.

For example, the processor 220 determines a location of a first focal spot in a target area to which ultrasound is to be initially-irradiated, and a location of a second focal spot in a target area to which ultrasound is to be irradiated after the first focal spot, such that the location of the second focal spot is the farthest from the location of the first focal spot. The processor 220 may determine a location of a third focal spot in the target area to which ultrasound is to be irradiated after the second focal spot, such that the location of the third focal spot is the farthest from the locations of the first and second focal spots.

When the processor 220 determines locations of focal spots in a target area to which ultrasounds are to be irradiated, a location that is the farthest from locations of focal spots in the target area to which ultrasounds have been previously-irradiated, may be an area in which tissues are necrosed or do not correspond to a lesion. In this example, the processor 220 determines another location that is farthest from the locations of the focal spots to which ultrasounds have been previously-irradiated. In other words, when it is difficult to secure a sufficient distance for cooling from the locations of the focal spots to which ultrasounds have been previously-irradiated, ultrasound is irradiated to the other farthest location after a sufficient cooling time interval.

Since the location of the second focal spot is the farthest from the location of the first focal spot, an influence of a thermal dose of the ultrasound irradiated to the first focal spot upon the second focal spot may be reduced or minimized. Furthermore, since the third focal spot is located the farthest from the locations of the first and second focal spots, an influence of the thermal dose of the ultrasound irradiated to the first and second focal spots upon the third focal spot may be reduced or minimized. Therefore, a cooling time interval between the irradiation of the ultrasound to the first focal spot and the irradiation of the ultrasound to the second focal spot, and a cooling time interval between the irradiation of the ultrasound to the second focal spot and the irradiation of the ultrasound to the third focal spot, may be reduced or minimized.

Since the focused ultrasound irradiation apparatus 200 sequentially irradiates ultrasounds to focal spots that are arranged to be away from each other as far as possible, an influence of a thermal dose due to a previous irradiation of ultrasound may be reduced or minimized. Thus, cooling time intervals between sequential irradiations of the ultrasounds may be reduced or minimized during an overall focused ultrasound irradiation process. Furthermore, normal tissues may be prevented from being necrosed by the thermal dose due to the previous irradiation of the ultrasound. As a result, an overall operation time of the focused ultrasound irradiation process may be reduced, and the focused ultrasound irradiation operation may be performed in consideration of safety and convenience of a patient.

The transducer 230 irradiates ultrasound based on the size of the focal spot, the locations of the focal spots, and the durations and the intensities of the ultrasound irradiations that are determined by the processor 220, and based on the image of the target area that is generated by the image generating unit 210. In more detail, the transducer 230 converts electric energy to ultrasound energy (e.g., HIFU energy), and focuses the ultrasound energy at the focal spots. In other words, the focused ultrasound irradiation apparatus 200 locates or moves the transducer 230 to be focused on a lesion to be cured. The transducer 230 performs an irradiation of the lesion by irradiating the ultrasound energy to the locations of the focal spots based on the intensities and the durations of the ultrasound irradiations, and the image of the target area.

The temperature monitoring unit 240 monitors thermal doses and temperatures of a target area and focal spots in the target area to which ultrasounds are to be irradiated. The processor 220 may determine the locations of the focal spots based on the size of the focal spot that is determined by the processor 220, and the temperatures and the thermal doses that are monitored by the temperature monitoring unit 240, such that each of the locations of the focal spots is the farthest from locations of focal spots in the target area to which ultrasounds have been previously-irradiated. For example, the processor 220 may refer to the thermal doses and the temperatures to determine the locations of the focal spots based on whether tissues outside the target area will be protected and whether tissues at a focal spot in the target area to which ultrasound is to be irradiated may be necrosed.

The communication unit 250 receives an irradiation plan from the irradiation plan generating apparatus 100 of FIG. 1 that is used by the focused ultrasound irradiation apparatus 200 to treat a target area. Based on the received irradiation plan, the processor 220 may determine a size of a focal spot in the target area, locations of focal spots in the target area, and durations and intensities of ultrasound irradiations.

In other words, the focused ultrasound irradiation apparatus 200 may receive a pre-generated irradiation plan via the communication unit 250, and perform an irradiation of a target area based on the received irradiation plan. Alternatively, the focused ultrasound irradiation apparatus 200 may determine a size of a focal spot in the target area, locations of focal spots in a target area, and durations and intensities of ultrasound irradiations based on the image of the target area that is generated by the image generating unit 210 in real time, and the thermal doses and the temperatures that are monitored by the temperature monitoring unit 240, to perform the irradiation. Alternatively, the focused ultrasound irradiation apparatus 200 may determine the size of the focal spot, the locations of the focal spots, and the durations and the intensities of the ultrasound irradiations by partially modifying the received irradiation plan based on the image of the target area and the temperatures and the thermal doses, to perform the irradiation.

Although FIG. 2 shows that the focused ultrasound irradiation apparatus 200 includes the temperature monitoring unit 240 and the communication unit 250, the focused ultrasound irradiation apparatus 200 is not limited thereto, and the focused ultrasound irradiation apparatus 200 may operate without including the temperature monitoring unit 240 and the communication unit 250. Therefore, the temperature monitoring unit 240 and the communication unit 250 may not be components of the focused ultrasound irradiation apparatus 200.

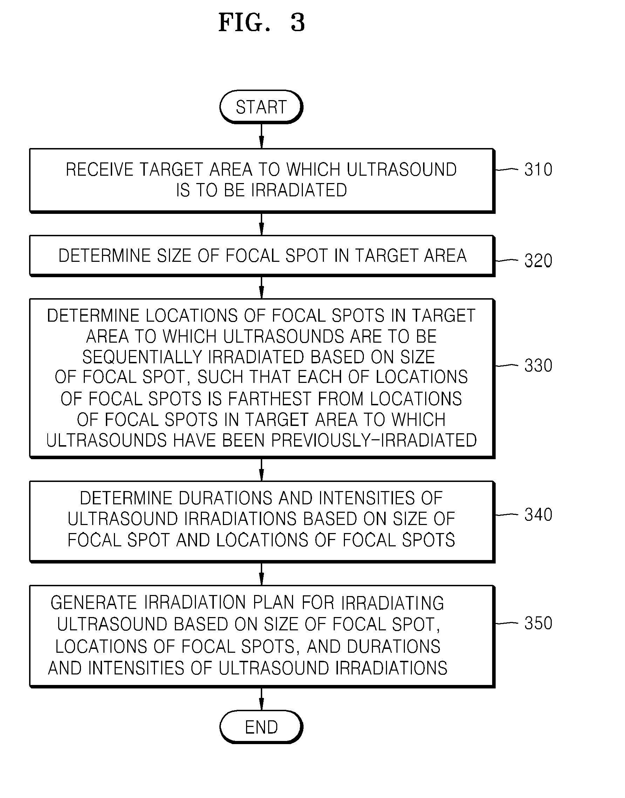

FIG. 3 is a flowchart view illustrating an example of a method of generating an irradiation plan. Referring to FIG. 3, the method of generating the irradiation plan includes operations that are chronologically performed by the irradiation plan generating apparatus 100 shown in FIG. 1. Therefore, even if omitted below, any of the descriptions given above regarding the irradiation plan generating apparatus 100 shown in FIG. 1 may also be applied hereto.

In an operation 310, the input interface 110 receives a target area to which ultrasound is to be irradiated. The target area corresponds to a lesion of a patient to be treated, and includes an area treatable by sequentially irradiating a single ultrasound during a single operation of the focused ultrasound irradiation apparatus 200 of FIG. 1. The target area may be displayed based on an image of the target area that is obtained via a CT apparatus, an MRI apparatus, and/or a diagnostic ultrasound system.

In an operation 320, the first determining unit 120 determines a size of a focal spot in the target area to which a single ultrasound is to be irradiated.

In an operation 330, the second determining unit 130 determines locations of focal spots in the target area to which ultrasounds are to be sequentially irradiated based on the size of the focal spot, such that each of the locations of the focal spots is the farthest from locations of focal spots in the target area to which ultrasounds have been previously-irradiated.

In an operation 340, the third determining unit 140 determines durations and intensities of ultrasound irradiations based on the size of the focal spot and the locations of the focal spots. In more detail, the third determining unit 140 determines a duration and an intensity of ultrasound irradiation to each of the locations of the focal that are determined by the second determining unit 130 with the size of the focal spot that is determined by the first determining unit 120.

In an operation 350, the plan generating unit 150 generates an irradiation plan for irradiating ultrasound based on the size of the focal spot, the locations of the focal spots, and the durations and the intensities of the ultrasound irradiations. The generated irradiating plan may be used for the focused ultrasound irradiation apparatus 200 to irradiate ultrasound. The irradiation plan indicates a method of irradiating ultrasound to a lesion of a patient, in the focused ultrasound irradiation apparatus 200.

For example, based on the size of the focal spot, the locations of the focal spots, and the durations and the intensities of the ultrasound irradiations, the plan generating unit 150 may generate the irradiation plan to include information regarding a path in which a transducer of the focused ultrasound irradiation apparatus 200 is to move via, e.g., a simulation. Furthermore, the plan generating unit 150 may generate the irradiation plan to include information regarding ultrasound to be irradiated by the transducer.

Since the irradiation plan generating apparatus 100 plans to sequentially irradiate ultrasounds to focal spots that are arranged to be away from each other as far as possible, an influence of a thermal dose due to a previous irradiation of ultrasound may be reduced or minimized. Thus, cooling time intervals between sequential irradiations of the ultrasounds may be reduced or minimized during an overall focused ultrasound process. Furthermore, normal tissues may be prevented from being necrosed by the thermal dose due to the previous irradiation of the ultrasound. As a result, an overall operation time of the focused ultrasound irradiation process may be reduced, and the focused ultrasound irradiation operation may be performed in consideration of safety and convenience of a patient.

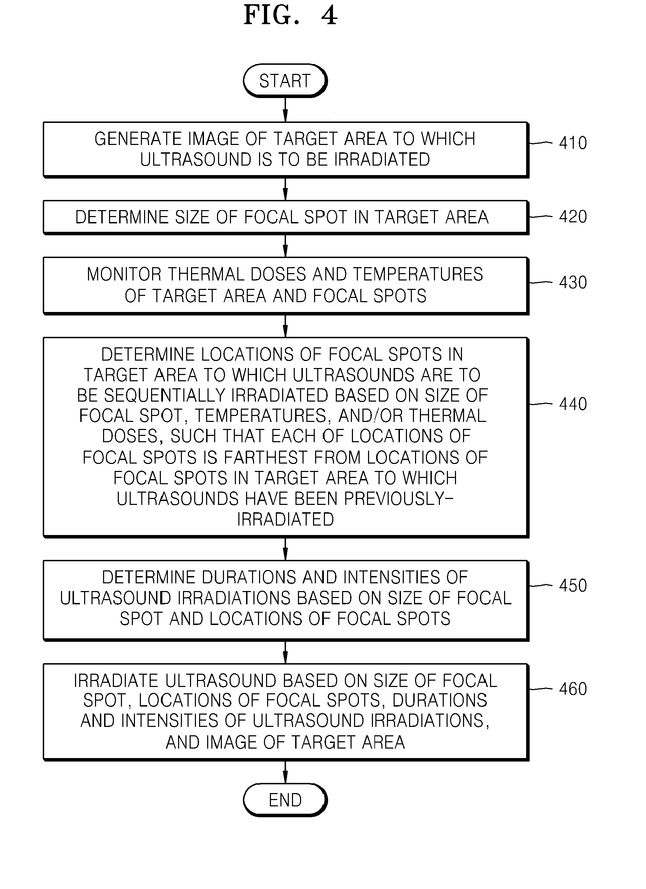

FIG. 4 is a flowchart view illustrating an example of focused ultrasound irradiation method. Referring to FIG. 4, the focused ultrasound irradiation method includes operations that are chronologically performed by the focused ultrasound irradiation apparatus 200 shown in FIG. 2. Therefore, even if omitted below, any of the descriptions given above regarding the focused ultrasound irradiation apparatus 200 shown in FIG. 2 may also be applied hereto.

In an operation 410, the image generating unit 210 generates an image of a target area to which ultrasound is to be irradiated. The target area corresponds to a lesion of a patient to be treated, and includes an area treatable by sequentially irradiating a single ultrasound during a single operation of the focused ultrasound irradiation apparatus 200. The image generating unit 210 may generate the image of the target area by using a CT apparatus, an MRI apparatus, and/or a diagnostic ultrasound system. However, the example of FIG. 4 is not limited thereto.

Furthermore, the image generating unit 210 may monitor an irradiation process of a target area in real time. For example, the image generating unit 210 may monitor whether normal tissues are protected, and whether tissues at a focal spot in the target area to which ultrasound is irradiated are necrosed. Furthermore, the image generating unit 210 may obtain images of the target area, and determine whether the irradiation process is completed.

In an operation 420, the processor 220 determines a size of a focal spot in the target area to which a single ultrasound is to be irradiated.

In an operation 430, the processor 220 monitors thermal doses and temperatures of the target area and focal spots to which ultrasounds are to be irradiated.

In an operation 440, the processor 220 determines locations of the focal spots in the target area to which ultrasounds are to be irradiated based on the size of the focal spot, the temperatures, and/or the thermal doses, such that each of the locations of the focal spots is the farthest from locations of focal spots in the target area to which ultrasounds have been previously-irradiated. For example, the processor 220 may refer to the thermal doses and the temperatures to determine the locations of the focal spots based on whether tissues outside the target area will be protected, and whether tissues at a focal spot in the target area to which ultrasound is to be irradiated may be necrosed.

In an operation 450, the processor 220 determines durations and intensities of ultrasound irradiations based on the size of the focal spot and the locations of the focal spots.

In an operation 460, the transducer 230 irradiates ultrasound based on the size of the focal spot, the locations of the focal spots, the durations and the intensities of the ultrasound irradiations, and the image of the target area. In more detail, the focused ultrasound irradiation apparatus 200 locates or moves the transducer 230 to be focused on a lesion to be cured. The transducer 230 performs an irradiation of the lesion by irradiating ultrasound energy (e.g., HIFU energy) to the locations of the focal spots based on the intensities and the durations of the ultrasound irradiations, and the image of the target area.

According to another example of a focused ultrasound irradiation method, the focused ultrasound irradiation apparatus 200 may irradiate ultrasound based on the irradiation plan, which is described by the method of FIG. 3.

Since the focused ultrasound irradiation apparatus 200 sequentially irradiates ultrasounds to focal spots that are arranged to be away from each other as far as possible, an influence of a thermal dose due to a previous irradiation of ultrasound may be reduced or minimized. Thus, cooling time intervals between sequential irradiations of the ultrasounds may be reduced or minimized during an overall focused ultrasound irradiation process. Furthermore, normal tissues may be prevented from being necrosed by the thermal dose due to the previous irradiation of the ultrasound. As a result, an overall operation time of the focused ultrasound irradiation process may be reduced, and the focused ultrasound irradiation operation may be performed in consideration of safety and convenience of a patient.

The units described herein may be implemented using hardware components and software components. For example, the hardware components may include microphones, amplifiers, bandpass filters, audio to digital convertors, and processing devices. A processing device may be implemented using one or more general-purpose or special purpose computers, such as, for example, a processor, a controller and an arithmetic logic unit, a digital signal processor, a microcomputer, a field programmable array, a programmable logic unit, a microprocessor or any other device capable of responding to and executing instructions in a defined manner. The processing device may run an operating system (OS) and one or more software applications that run on the OS. The processing device also may access, store, manipulate, process, and create data in response to execution of the software. For purpose of simplicity, the description of a processing device is used as singular; however, one skilled in the art will appreciated that a processing device may include multiple processing elements and multiple types of processing elements. For example, a processing device may include multiple processors or a processor and a controller. In addition, different processing configurations are possible, such a parallel processors..

The software may include a computer program, a piece of code, an instruction, or some combination thereof, that independently or collectively instructs or configures the processing device to operate as desired. Software and data may be embodied permanently or temporarily in any type of machine, component, physical or virtual equipment, computer storage medium or device, or in a propagated signal wave capable of providing instructions or data to or being interpreted by the processing device. The software also may be distributed over network coupled computer systems so that the software is stored and executed in a distributed fashion. The software and data may be stored by one or more computer readable recording mediums. The computer readable recording medium may include any data storage device that can store data which can be thereafter read by a computer system or processing device. Examples of the non-transitory computer readable recording medium include read-only memory (ROM), random-access memory (RAM), CD-ROMs, magnetic tapes, floppy disks, optical data storage devices. Also, functional programs, codes, and code segments that accomplish the examples disclosed herein can be easily construed by programmers skilled in the art to which the examples pertain based on and using the flow diagrams and block diagrams of the figures and their corresponding descriptions as provided herein.

As a non-exhaustive illustration only, a device described herein may refer to mobile devices such as a cellular phone, a personal digital assistant (PDA), a digital camera, a portable game console, and an MP3 player, a portable/personal multimedia player (PMP), a handheld e-book, a portable laptop PC, a global positioning system (GPS) navigation, a tablet, a sensor, and devices such as a desktop PC, a high definition television (HDTV), an optical disc player, a setup box, a home appliance, and the like that are capable of wireless communication or network communication consistent with that which is disclosed herein.

A number of examples have been described above. Nevertheless, it will be understood that various modifications may be made. For example, suitable results may be achieved if the described techniques are performed in a different order and/or if components in a described system, architecture, device, or circuit are combined in a different manner and/or replaced or supplemented by other components or their equivalents. Accordingly, other implementations are within the scope of the following claims.

## Claims

1. A method of generating an irradiation plan for irradiating ultrasound, the method comprising:
determining a size of a focal spot in an area to which ultrasound is to be irradiated;
determining locations of focal spots in the area to which ultrasounds are to be sequentially irradiated based on the size of the focal spot, such that each of the locations of the focal spots is farthest from locations of focal spots in the area to which ultrasounds have been previously-irradiated;
determining durations and intensities of ultrasound irradiations based on the size of the focal spot and the locations of the focal spots; and
generating the irradiation plan for irradiating ultrasound based on the size of the focal spot, the locations of the focal spots, and the durations and the intensities of the ultrasound irradiations.

2. The method of claim 1, further comprising:
determining a location of a first focal spot in the area to which ultrasound is to be initially-irradiated; and
determining a location of a second focal spot in the area to which ultrasound is to be irradiated after the first focal spot, such that the location of the second focal spot is farthest from the location of the first focal spot,
preferably comprising:
determining a location of a third focal spot in the area to which ultrasound is to be irradiated after the second focal spot, such that the location of the third focal spot is farthest from the locations of the first and second focal spots.

3. The method of claim 2, comprising:
determining locations of successive focal spots to be away from each other as far as possible to minimize an influence of a thermal dose of ultrasound irradiated to the first focal spot upon the second focal spot and to minimize a cooling time interval between irradiation of the ultrasound to the first focal spot and irradiation of ultrasound to the second focal spot.

4. The method of any of the preceding claims, further comprising:
estimating thermal doses and temperatures of the area and the focal spots; and
determining the locations of the focal spots further based on the temperatures and the thermal doses.

5. The method of any of the preceding claims, further comprising:
outputting the irradiation plan to the focused ultrasound irradiation apparatus.

6. A focused ultrasound irradiation method for irradiating ultrasound based on the irradiation plan, generated by the method of any one of the preceding claims, the focused ultrasound irradiation method comprising:
generating an image of the area to which ultrasound is to be irradiated; and
irradiating ultrasound based on the size of the focal spot, the locations of the focal spots, the durations and the intensities of the ultrasound irradiations included in the irradiation plan and the generated image.

7. The method of claim 5, further comprising:
monitoring thermal doses and temperatures of the area and the focal spots; and
determining the locations of the focal spots further based on the temperatures and the thermal doses.

8. A non-transitory computer readable storage medium storing a program comprising instructions to cause a computer to implement the method of any of the preceding claims.

9. An irradiation plan generating apparatus configured to generate an irradiation plan for irradiating ultrasound, the irradiation plan generating apparatus comprising:
a first determining unit configured to determine a size of a focal spot in an area to which ultrasound is to be irradiated;
a second determining unit configured to determine locations of focal spots in the area to which ultrasounds are to be sequentially irradiated based on the size of the focal spot, such that each of the locations of the focal spots is farthest from locations of focal spots in the area to which ultrasounds have been previously-irradiated;
a third determining unit configured to determine durations and intensities of ultrasound irradiations based on the size of the focal spot and the locations of the focal spots; and
a plan generating unit configured to generate the irradiation plan for irradiating ultrasound based on the size of the focal spot, the locations of the focal spots, and the durations and the intensities of the ultrasound irradiations.

10. The irradiation plan generating apparatus of claim 9, wherein the second determining unit is further configured to:
determine a location of a first focal spot in the area to which ultrasound is to be initially-irradiated;
determine a second focal spot in the area to which ultrasound is to be irradiated after the first focal spot, such that the location of the second focal spot is farthest from the location of the first focal spot; and
determine a location of a third focal spot in the area to which ultrasound is to be irradiated after the second focal spot, such that the location of the third focal spot is farthest from the locations of the first and second focal spots,
wherein the determining unit is preferably configured to:
determine locations of successive focal spots to be away from each other as far as possible to minimize an influence of a thermal dose of ultrasound irradiated to the first focal spot upon the second focal spot and to minimize a cooling time interval between irradiation of the ultrasound to the first focal spot and irradiation of ultrasound to the second focal spot.

11. The irradiation plan generating apparatus of claim 8, 9 or 10, further comprising:
an estimating unit configured to estimate thermal doses and temperatures of the area and the focal spots,
wherein the second determining unit is further configured to determine the locations of the focal spots further based on the temperatures and the thermal doses.

12. The treatment plan generating apparatus of claims 8 through 11, further comprising:
an output interface configured to output the irradiation plan to the focused ultrasound irradiation apparatus.

13. A focused ultrasound irradiation apparatus including the irradiation plan generating apparatus of any one of claims 9 through 12, the focused ultrasound irradiation apparatus comprising:
an image generating unit configured to generate an image of the area to which ultrasound is to be irradiated; and
a transducer configured to irradiate ultrasound based on the size of the focal spot, the locations of the focal spots, the durations and the intensities of the ultrasound irradiations included in the irradiation plan, and the image of the area.

14. The focused ultrasound irradiation apparatus of claim 13, further comprising:
a temperature monitoring unit configured to monitor thermal doses and temperatures of the area and the focal spots,
wherein the second determining unit is further configured to determine the locations of the focal spots further based on the temperatures and the thermal doses.

15. The focused ultrasound irradiation apparatus of claim 13 or 14, further comprising:
a communication unit configured to receive the irradiation plan to treat the area from an external device.
